# EUROPEAN PATENT SPECIFICATION

(11) **EP 0 238 207 B1**
(45) Date of publication and mention of the grant of the patent: **26.05.1993**
(21) Application number: 87301336.1
(22) Date of filing: 17.02.1987
(51) Int. Cl.: A61K 31/635

(54) **Bactericidal mixtures**
Bakterientötende Gemische
Mélanges bactéricides

(30) Priority: 18.02.1986 GB 8603962
(43) Date of publication of application: 23.09.1987
(73) Proprietor: PITMAN-MOORE INC., Lake Forest Illinois 60045 (US)
(72) Inventor: White, Geoffrey, Berkhamsted Hertfordshire (GB)
(74) Representative: Matthews, Derek Peter

(56) References cited:
- EP-A- 0 051 879

## Description

The present invention relates to synergistic compositions of a sulphonamide and a 5-arylmethyl-2,4-diaminopyrimidine and their use in the treatment of microbial infections, particularly those of veterinary significance.

European Patent Application 51879 discloses the compound 2,4-diamino-5-(8-dimethylamino-7-methyl-5-quinolylmethyl)pyrimidine as having good in vitro antibacterial activity against a range of organisms. However it has been found to be ineffective in treating acute salmonellosis (S.dublin) in calves when administered alone at dosages of up to 6 mg/kg.

It has been reported (G. White et. al.,Research in Veterinary Science, 1981, 31, 27-31) that a combination of trimethoprim and sulphadiazine is effective in treating salmonellosis in calves. However, this combination, in order to be effective, must be administered on a daily basis. Moreover, the oral bioavailability of trimethoprim in cattle older than about six weeks is very poor. Thus oral administration, even in high doses, does not result in significant concentrations of trimethoprim in body fluids and therefore trimethoprim products are not administered orally to cattle older than six weeks.

It has now been discovered that a combination of 2,4-diamino-5-(8-dimethylamino-7-methyl-5-quinolylmethyl)pyrimidine and sulphadimidine is highly effective in the treatment of salmonellosis and, moreover, the pharmacokinetics of the combination are such that it may be administered much less frequently than is the case with the trimethoprim/sulphadiazine combination. In addition, the oral bioavailability is so much better than that of the trimethoprim/sulphadiazine combination referred to above that these novel combinations may be administered orally to cattle of all ages to provide effective treatment.

Accordingly, the present invention provides a composition containing 2,4-diamino-5-(8-dimethylamino-7-methyl-5-quinolylmethyl)pyrimidine and sulphadimidine or salts thereof and optionally a pharmaceutically acceptable carrier therefor.

Combinations of the present invention are synergistic. Thus for example, in the treatment of calves infected with Salmonella, neither component when used alone produces significant improvements in mortality rates, whereas combined treatments using half or less of the individual doses do result in improvements.

The compositions may be adapted for administration by routes well known to those skilled in the veterinary and formulation sciences. The pyrimidine and sulphadimidine may be present in the compositions as salts or in free unsalted form. It is often convenient for one of the pyrimidine and sulphadimidine to be present as a salt, for example sodium sulphadimidine or an acid addition salt of the pyrimidine, for example formed from lactic, citric or formic acids, whilst the other component is present in free unsalted form (see for example GB-B-1176395, 1347472 and 1469521). Thus, for example, they may be adapted for oral, parenteral, topical, intravaginal or intrarumenal administration.

Compositions suitable for oral administration include drenches (oral liquid dosing), which may be solutions or suspensions; tablets, boluses, pastes, or in-feed preparations in the form of powders, granules or pellets. Preferred orally administrable compositions include boluses.

Alternatively, the compositions may be adapted to be administered parenterally by sub-cutaneous, intramuscular or intravenous injection of a sterile solution or suspension, by implantation or as an intramammary injection whereby a suspension or solution is introduced into the udder via the teat.

Compositions suitable for topical administration include creams, ointments or sprays applied to the skin, and intravaginal compositions include pessaries, creams and foams.

For intrarumenal injection, the compositions of the invention may be solutions or solid or microcapsule suspensions. Typically the compositions are similar to the oral liquid preparations or parenteral preparations described herein. Such compositions are injected directly into the rumen, usually through the side of the animal, for example by a hypodermic syringe and needle or by an automatic injection device capable of giving single or multiple doses. In view of the good bioavailability of the combination of invention, the intrarumenal route of administration is a preferred one.

The pharmaceutically acceptable carriers present in the compositions of the present invention are materials recommended for the purpose of administering the medicament. These may be liquid, solid or gaseous materials, which are otherwise inert or medically acceptable and are compatible with the active ingredient.

For oral administration, fine powders or granules will contain diluting, for example lactose, calcium carbonate, calcium phosphate, mineral carriers, etc., dispersing and/or surface active agents, for example polysorbates such as Tweens or Spans, and may be presented in a drench, in water or in a syrup, in a bolus, paste, or in a feed preparation, in capsules or sachets in the dry state or in a non-aqueous suspension, or in a suspension in water or syrup. Where desirable or necessary, preserving, suspending, thickening or emulsifying agents can be included. If intended for oral use, a bolus will be provided with retention means to inhibit regurgitation, for example it may be weighted with a heavy density material such as iron or tungsten or the like or may be retained by its shape, for example by wings which spring after administration. Boluses may contain disintegrating agents such as maize starch or calcium or sodium methyl celluloses, hydroxypropylmethyl cellulose, guar based vegetable gums, sodium alginates or sodium starch glycolates; granulating or binding agents such as starch in the form of mucilage, starch derivatives, such as "Snow Flake", cellulose derivatives such as talc, calcium stearate, methyl cellulose, gelatin or polyvinylpyrrolidone; and/or lubricating agents, such as magnesium stearate or stearic acid.

For parenteral administration, the compounds may be presented in sterile injection solutions which may contain antioxidants or buffers, or as injectable suspensions. Suitable solvents include water, in the case of suspensions, and organic solvents such as dimethylformamide, dimethylacetamide, diethylacetamide, ethyl lactate, ethyl akate, dimethylsulphoxide, alcohols, e.g. ethanol, glycols, e.g. ethylene glycol, propylene glycol, butylene glycol and hexamethylene glycol, polyethylene glycols containing 2 to 159 ethylene glycol monomer units and having average molecular weights from about 90 to 7500, glycerin formal, glycofural, glycerol, isopropylmyristate N-methylpyrrolidone, 2-pyrrolidone polyethylene glycoethers of tetrahydrofurfuryl alcohol and diethylene glycol, and fixed and neutral oils, for example fractionated coconut oil. One formulation found to be particularly advantageous for the treatment of calf salmonellosis by intra-muscular injection comprises an 18% 1:5 (pyrimidine:sulphonamide) suspension in fractionated coconut oil (Miglyol). This formulation has the advantage that it does not cause pain at the site of injection.

Other compounds which may be included are, for example, medically inert ingredients, e.g. solid and liquid diluents such as lactose, glucose, starch or calcium phosphate for tablets, boluses or capsules; olive oil or ethyl oleate for soft capsules; and water or vegetable oil for suspensions or emulsions; lubricating agents such as talc or magnesium sterarate; gelling agents such as colloidal clays; thickening agents such as gum tragacanth or sodium alginate; dedusting agents such as liquid paraffin, fixedoils and surfactants and other therapeutically acceptable accessory ingredients such as humectants, preservatives, buffers, and antioxidants which are useful as carriers in such formulations.

When desired, other medicaments and/or nutrients such as vitamins may also be included.

Alternatively the active compound may be presented in a pure form as an effective unit dosage, for instance, compressed as a tablet.

The compositions of the present invention can be prepared by the admixture of the active compounds with a pharmaceutically acceptable carrier. Other ingredients, for example the conventional pharmaceutical excipients described above may be added as required.

In view of the good oral bioavailability of the combination of the invention, the oral route of administration is a preferred one.

The doses and ratios of the components of the composition are chosen such that there is provided at the site of infection a concentration of each component sufficient to exert a synergistic effect. In general the active ingredients are present in a ratio of between 1:1 and 1:20 pyrimidine:sulphonamide. Suitably, combinations for parenteral use are those having the ratio of 1:5 pyrimidine:sulphonamide. For oral administration suitably the active ingredients are present in a ratio of between 1:4 and 1:10 pyrimidine:sulphonamide, preferably 1:9.

The invention also provides a method of treatment of bacterial infections in animals, particularly those caused by Salmonella sp., Pasteurella haemolytica and Streptococcus suis, which method comprises the administration of an effective amount of a composition as hereinbefore defined. In general the dosages are such that the amount of the pyrimidine administered per day is in the range 0.5 mg/kg bodyweight to 10.0 mg/kg, suitably 1.0 mg/kg to 10mg/kg and preferably 1.0 mg/kg to 4.5 mg/kg and the amount of sulphonamide administered per day is in the range 1 mg/kg bodyweight to 100 mg/kg bodyweight, preferably 5 mg/kg bodyweight to 40 mg/kg bodyweight, although the optimal dosages will vary according to the route of administration. Thus when administered to pigs by means of an in-feed composition, the active ingredients typically are administered in a ratio of 1:5 pyrimidine: sulphonamide at a concentration in the feed of approximately 100 ppm.

When administered parenterally, for example by intramuscular injection, the composition typically is administered at dosages of 10-30 mg/kg active ingredients wherein the ratio of pyrimidine:sulphonamide is 1:5.

When administered orally to cattle in a bolus formulation, a ratio of 1:9 is preferred. A once-in-two-days bolus formulation typically contains 1mg/kg to 8mg/Kg of the pyrimidine, preferably 4 mg/kg; and 9mg/kg to 72mg/kg of sulphadimidine, preferably 36mg/kg of sulphadimidine. A once-in-five-days bolus formulation typically contains 6 mg/kg to 16 mg/kg of the pyrimidine, preferably 8 mg/kg; and 54 mg/kg to 144 mg/kg of sulphadimidine, preferably 72 mg/kg of sulphadimidine.

Bolus formulations will normally contain a unit dose of 0.5g to 3g of pyrimidine,for example 0.75g or 1.5g of pyrimidine, and 5g to 30g of sulphadimidine, for example 7.5g or 1.5g of sulphadimidine.

The compositions of the present invention are particularly advantageous in the treatment of livestock species for example cattle, sheep, goats, horses or pigs.

The invention will now be illustrated in greater detail by means of examples. In the following examples 2,4-diamino-5-(8-dimethylamino-7-methyl-5-quinolylmethyl) pyrimidine is referred to as Compound A, sulphadimidine is referred to as SDD and trimethoprim as TMP.

### Example 1

### A Comparison of the Therapeutic Efficacies of Trimethoprim and Compound A in Experimental Bovine Salmonellosis Materials and Methods

The technique of infecting calves orally with Salmonella dublin and the use of the model for chemotherapy studies has been described by White et al, 1981A and 1981B. Calves approximately 3 weeks old received 10¹⁰ S.dublin strain M738, and treatment was given once daily beginning on the second day (Day 2) after infection. The experimental design is shown in Table 1. The duration of treatment was 3 days.

For injection, an aqueous 33.3% w/v solution of sodium sulphadimidine was used and compounds A and TMP were dissolved in water as the hydrochloride or lactate respectively at 10 or 20 mg/ml. Injections were given on opposite sides of the animal subcutaneously. For oral administration either sulphadimidine (SDD) powder plus A or SDD powder plus TMP were enclosed together in a 3.5 ml capsule, which was administered by a balling gun.

Regular bacteriological examination of rectal swabs before and after infection was carried out, and all carcases were examined bacteriologically. Serum samples were examined for concentrations of TMP and Compound A by bio-assay using B.pumilus as test organism, and for SDD by the Bratton-Marshall method.

Rectal temperatures were taken daily up to 10 days after infection.

### Results and Discussion

In this experiment it was considered justifiable to omit the inclusion of untreated groups, in order to obtain maximal group sizes of treated calves. White et al (1981A) reported the consistently high mortality (86%) in this disease model which followed the standard challenge procedure using a large number of untreated calves. The probability of obtaining any particular mortality figure can therefore be predicted providing that the conditions of the experiment are maintained as constant as possible. The mortality results with both TMP plus SDD (8 out of 15) and Compound A plus SDD (3 out of 15) were significantly better than the expected mortality in the absence of treatment (p <0.01), but the result with Compound A was clearly superior. Further deaths occured in both groups after Day 10, but these were primarily due to a TMP-resistant strain of S.typhimurium which was present in the calves at the time of purchase, and which became widely distributed during the therapeutic period.

### References

White, G., Piercy, D.W.T., Clampitt, R.G., Morgan, R.J.I., West, B. (1981A) Res. Vet. Sci. 31, 19-26.

White, G., Piercy, D.W.T., Gibbs, H.A. (1981B) Res. Vet. Sci. 31, 27-31.

**Table 1**

| Group | Day | Daily Dose (mg/kg) | | | No. of calves | Cumulative mortality | | | | | | |
|---|---|---|---|---|---|---|---|---|---|---|---|---|
| | | TMP | Compound A | SDD | | D4 | D5 | D6 | D7 | D8 | D9 | D10 |
| A | 1 | 4 | - | 20 | 15 | 1 | 1 | 2 | 4 | 5 | 7 | 8 |
| | 2^{o}3 | 2 | - | 10 | | | | | | | | |
| B | 1 | - | 4 | 20 | 15 | 0 | 1 | 1 | 1 | 1 | 1 | 3 |
| | 2^{o}3 | - | 2 | 10 | | | | | | | | |

### Example 2

### Investigation of synergy between Compound A and sulphadimidine in the treatment of experimental bovine salmonellosis by repeated daily injections.

### Introduction

A method of consistently reproducing high mortality (86%) in young calves infected by the oral route with a culture of Salmonella dublin, and the use of this disease model in experimental chemotherapy, has already been described by White et al (1981a and 1981b). In planning the investigation of possible synergy between Compound A and sulphadimidine (SDD), it was decided that seven treatment groups (including an untreated control group) of eight calves each would be necessary, but because of the impossibility of housing this required total number of calves (56) at one time in the available isolation accommodation, it was planned to carry out two duplicate experiments each using seven groups of four calves.

### Materials and Methods

### In vitro studies

Estimation of the minimum inhibitory concentrations (MICs) of Compound A and SDD, alone and together, was carried out by a surface growth inhibition method, using a "checkerboard" two-fold dilution series, with Compound A and SDD ranges of 0.512 to 0.002 g/ml and 256 to 1µg/ml respectively. Thus, including the control plates, a total of 100 different combinations were used. Dilutions were made in Oxoid Isosensitest Agar with plate inoculation by an automatic inoculator using a 2µl inoculum yielding semi-confluent growth over a disc approx. 7 mm diameter. End points were judged to be the lowest concentrations completely or almost completely inhibiting growth.

### Formulations

1) "Sodium Sulfamethazine Injectable Solution 25%" - Cyanamid. This commercial preparation contained 250 mg/ml of sodium SDD, equivalent to 232 mg/ml of SDD.
2) Aqueous solution of SDD, 100 mg/ml in dilute NaOH.
3) Aqueous solution of Compound A, 20 mg/ml as the hydrochloride.

### Experimental Design

| Group | Daily dose (mg/kg) | |
|---|---|---|
| | Compound A | SDD |
| 1 | - | 200/100* |
| 2 | - | 30 |
| 3 | 6 | - |
| 4 | 3 | 15 |
| 5 | 2 | 10 |
| 6 | 1.3 | 6.7 |
| 7 | | |

| | | |
|---|---|---|
| * First dose 200 mg/kg, subsequent doses 100 mg/kg using the Cyanamid formulation, derived from the recommended rates of "2ml for 2.27kg (5lb)" with subsequent doses at half this level. | | |

### Procedure

In the first experiment (Experiment I), 28 market-purchased calves (approximately one week old) were held for 18 days before infection, during which time rectal swabs collected on three occasions from each calf were negative for salmonella. On the day of infection, Day 0, each calf received an oral dose of 50 ml of a log-phase culture of Salmonella dublin strain M738 diluted to contain an estimated 10¹⁰ organisms, using the published technique (White et al 1981a). A viable count on the inoculum was carried out.

On the basis of Day 0 (am) bodyweights the calves were randomly allocated into 7 groups of 4. When the calves were infected at 2 pm on Day 0 it was decided that the severe respiratory symptoms exhibited by one calf (allocated to the control group) made it advisable to eliminate this calf from the experiment.

In the second experiment (Experiment II), 31 calves were used in order to replace three treated calves in Experiment I which had died within 24 hours of first treatment. Using Day 0 bodyweights, 3 calves of identical weights to the day 0 weights of the 3 eliminated calves were allocated to the appropriate groups, and the remaining 28 were randomly allocated to the 7 groups as above. As in Experiment I, the calves were shown to be salmonella-free before experimental infection.

Administration of compounds in all cases was by subcutaneous injection, using different sides of the animal for Compound A and SDD when both were given in groups 4, 5 and 6. The first of five consecutive daily injections was given 43 hours after injection, the other following at 24 hour intervals.

Blood samples for assay of sulphonamide by the Bratton-Marshall method and for Compound A by bio-assay were collected before and 24 hours after the first injection, 2, 5 and 24 hours after the second injection, 24 hours after the fourth injection and 2, 5 and 24 hours after the fifth injection.

### Results

### 1. Inoculum viable counts

The individual calf inocula in the two Experiments I and II contained 7.5 x 10⁹ and 9.5 x 10⁹ organisms respectively.

### 2. In vitro sensitivity results

The MICs of Compound A, SDD, alone and in various proportions, are shown in Table 2.

### Discussion

The total control mortality of 6 out of 7 (Table 3) did not differ significantly from that previously experienced with a large number of untreated calves (86.4% - White et al, 1981a). In group 1, dosed with the commercially recommended rate for SDD alone, the final mortality rate of 5 out of 8 also lacked significance, group mortalities of up to 5 out of 8 being expected with a total probability of 0.09 in untreated calves. The three surviving calves in this group showed a very slow clinical recovery and none had regained its Day 0 bodyweight by Day 21. In groups 2 and 3 treated with 30 mg/kg of SDD alone and 6 mg/kg of Compound A alone respectively, identical non-significant improvements in mortality rates of 5 out of 8 were recorded.

In the groups treated with both Compound A and SDD in 1:5 ratio, the mortality rate of 4 out of 7 at the lowest dose levels (Group 6, 1.3 plus 6.7 mg/kg)was of doubtful significance (p=0.06) in comparison with untreated controls, but the mortality rates in the two group (4 and 5) receiving higher doses were significantly better (for up to 3 out of 8, p = 0.002 and for 0 out of 8, p = 0.0001). The deaths of 3 calves at the highest doses ( 3mg/kg and 15 mg/kg of Compound A and SDD) in groups 4 was surprising in view of the absence of deaths in Group Two. The 3 deaths occurred at a late stage (Days 10 and 11) and appeared to be due to relapses as the calves had previously appeared to be responding well to treatment. By Day 21 in both experiments, most of the surviving calves were clinically normal and were gaining weight rapidly.

It is considered that the fact that neither treatment with Compound A nor SDD alone produced significant improvements in mortality rates, whereas combined treatments using half or less of the individual doses did so, is a clear demonstration that the synergy between Compound A and SDD which is evident in vitro (Table 2) is also applicable in vivo. In the case of SDD alone in Group 1, the daily dose rates were 10 to 20 times higher than those of SDD in Group 5, when Compound A was also given, but the higher dose schedule was therapeutically ineffective, in spite of the fact that serum concentrations of SDD were apparently greater than the in vitro MIC for SDD alone (128 µg/ml) throughout the dosing period. In group 3 (Compound A alone at 6 mg/kg), serum concentrations of Compound A were also higher than the individual MIC of Compound A of 0.256 µg/ml, but this dose rate was likewise ineffective.

**TABLE 2**

| Minimum Inhibitory Concentrations of Compound A and SDD, alone and together in various proportions for S. dublin strain M738. | | | | | | | | | | |
|---|---|---|---|---|---|---|---|---|---|---|
| | SDD µg/ml | | | | | | | | | |
| | 0 | 1 | 2 | 4 | 8 | 16 | 32 | 64 | 128 | 256 |
| 0.512 | - | - | - | - | - | - | - | - | - | - |
| 0.256 | - | - | - | - | - | - | - | - | - | - |
| 0.128 | + | - | - | - | - | - | - | - | - | - |
| 0.064 | + | + | - | - | - | - | - | - | - | - |
| 0.032 | + | + | + | - | - | - | - | - | - | - |
| Compound A µg/ml 0.016 | + | + | + | + | + | - | - | - | - | - |
| 0.008 | + | + | + | + | + | + | - | - | - | - |
| 0.004 | + | + | + | + | + | + | + | - | - | - |
| 0.002 | + | + | + | + | + | + | + | - | - | - |
| 0 | + | + | + | + | + | + | + | + | - | - |
| - = no growth at 24 hours + = growth at 24 hours | | | | | | | | | | |

**TABLE 3**

| Mortality pattern | | |
|---|---|---|
| Group | Treatment | Mortality |
| 1 | SDD 200/100 | 5 out of 8 |
| 2 | SDD 30 | 5 out of 8 |
| 3 | Compound A | 5 out of 8 |
| 4 | Compound A + SDD 3 + 15 | 3 out of 8 |
| 5 | Compound A + SDD 2 + 10 | 0 out of 7 |
| 6 | Compound A + SDD 1.3 + 6.7 | 4 out of 7 |
| 7 | None | 6 out of 7 |

### Example 3

### Drench or Intrarumenal Injection

Here the suspended solids content, and hence the viscosity, of the finished preparation is reduced by taking the sulphonamide into solution as its sodium salt. The salt is produced in situ by the addition of sodium hydroxide.

Because formulation is aqueous, methyl hydroxybenzoate is included as a preservative against microbiological contamination.

Polysorbate 80 is included to wet the suspended Compound A and improve dispersion characteristics.

Optionally, methyl cellulose may be incorporated to increase the viscosity of the finished product and maintain compound A in uniform suspension.

| | % w/v |
|---|---|
| Compound A (in fine powder form) | 3.0g |
| Sulphadimidine | 27.0g |
| Sodium Hydroxide | 3.5g or qs to produce pH 10-11 |
| Methyl Hydroxybenzoate | 0.1g |
| Polysorbate 80 | 0.1g |
| Purified Water | to 100.0 ml |

Suspend the Sulphadimidine in approximately 50 ml water. Add sodium hydroxide as 30% solution. Adjust the pH of the resulting solution to approximately 11.0 with further Sodium Hydroxide solution.

Disperse the Compound A and methyl hydroxybenzoate in a solution of the Polysorbate 80 in approximately 20 ml Water. Add this to the solution of Sulphadimidine Sodium and dilute to volume with Purified Water.

### Example 4

### Intramuscular Injection

| | |
|---|---|
| Compound A (in fine powder form) | 3.0g |
| Sulphadimidine(in fine powder form) | 15.0g |
| Fractionated Coconut Oil | to 100.ml |

Sterilise the Compound A and Sulphadimidine by heating at 160 °C for 1 hour. Under aseptic conditions add the drugs to the Fractionated Coconut Oil, previously sterilised by filtration through a membrane filter.

Mill the suspension to disperse aggregates, fill aseptically into vials and seal.

In this presentation both drugs remain in suspension in the vehicle.

### Example 5

### Granules for In-Feed Medication

| | |
|---|---|
| Compound A | 1.0g |
| Sulphadimidine | 5.0g |
| Povidone | 1.0g |
| Lactose | 93.0g |
| Aqueous alcohol mixture | qs. |

Mix together the Compound A, Sulphadimidine and Lactose. To this is added the aqueous alcohol which contains the dissolved Povidone. Sufficient aqueous alcohol is added to produce a moist mass. This mass is passed through a sieve to produce granules which are dried.

The Povidone (polyvinylpyrrolidone) is included as a binding agent.

### Example 6

### Oral Paste

| | |
|---|---|
| Compound A | 4.0g |
| Sulphadimidine | 20.0g |
| Xanthan Gum | 0.5g |
| Methyl Hydroxybenzoate | 0.1 g |
| Polysorbate 80 | 0.1g |
| Purified Water | to 100.0 ml |

Dissolve the Polysorbate 80 and Methyl Hydroxybenzoate in the bulk of the water. Add and disperse the Xanthan Gum and allow to swell. Add and disperse the Compound A and Sulphadimidine and dilute to volume.

The rationale for this formulation is similar to that for the Oral Drench but both drugs remain in suspension since high viscosity is a feature of the product. Xanthan Gum also contributes to thickening the product.

### Example 7

### Ointment

| | |
|---|---|
| Compound A | 2.0g |
| Sulphadimidine | 10.0g |
| White Soft Paraffin | 88.0g |

Melt the White Soft Paraffin at 60 °C. Add and disperse the Compound A and Sulphadimidine and allow to cool. Fill into collapsible metal tubes.

### Example 8

### Injectable solution

Each 100ml contains:

| | |
|---|---|
| Compound A | 3g |
| Sulphadimidine | 15g |
| Glycofural | q.s. 100 ml |

The components are mixed together and gently heated until dissolution. The resulting solution is cooled and stored under nitrogen and filled into ampoules or glass vials as required.

### Example 9

### Tablet

Each tablet contains:

| | |
|---|---|
| Compound A | 20mg |
| Sulphadimidine | 100mg |
| Sodium Starch Glycollate | 10mg |
| Microcrystalline Cellulose | 20 mg |
| Dicalcium Phosphate | 43.1 mg |
| Polyvinylpyrrolidone (Povidone K30) | 6.4mg |
| Magnesium Stearate | 0.5mg |

The first five ingredients are mixed together and granulated with a 15% solution of the polyvinylpyrrolidone in 50% aqueous alcohol. The resultant granules are dried, blended with magnesium stearate and compressed on a Manesty rotary punch to give 200 mg tablets.

## Claims (Claims for the following Contracting State(s): BE, CH, DE, FR, GB, IT, LI, LU, NL, SE)

1. A composition comprising the compounds 2,4-diamino-5-(8-dimethylamino-7-methyl-5-quinolylmethyl)pyrimidine and sulphadimidine, or salts thereof.

2. A composition according to claim 1 wherein the said compounds are present in a ratio of between 1:1 and 1:20.

3. A composition according to claim 1 for parenteral administration wherein the compounds are present in the ratio of 1:5.

4. A bolus or drench composition according to claim 1 wherein the compounds are present in the ratio of 1:9.

5. A paste or in-feed composition according to claim 1 wherein the compounds are present in the ratio of 1:5.

6. A composition according to any one of claims 1 to 5 for use in the treatment of bacterial infections in animals.

7. A composition according to claim 6 for use in the treatment of bacterial infections in animals by oral administration.

8. A composition according to claim 6 for use in the treatment of bacterial infections in animals by intraruminal administration.

9. A composition according to claim 1 in the form of a bolus which contains from 1 to 8 mg/kg of 2,4-diamino-5-(8-dimethylamino-7-methyl-5-quinolylmethyl)pyrimidine and from 9 to 72 mg/kg of sulphadimidine.

10. A composition according to claim 1 in the form of a bolus which contains from 6 to 16 mg/kg of 2,4-diamino-5-(8-dimethylamino-7-methyl-5-quinolylmethyl)pyrimidine and from 54 to 144 mg/kg of sulphadimidine.

11. A composition according to claim 1 in the form of a bolus which contains 8 mg/kg of 2,4-diamino-5-(8-dimethylamino-7-methyl-5-quinolylmethyl)pyrimidine and 72 mg/kg sulphadimidine.

12. A composition which comprises the compounds 2,4-diamino-5-(8-dimethylamino-7-methyl-5-quinolylmethyl)pyrimidine and sulphadimidine for use in the manufacture of a medicament.

13. A composition according to claim 12 which comprises the compounds for use in the manufacture of a medicament for oral administration.

14. A composition according to claim 12 which comprises the compounds for use in the manufacture of a medicament for intrarumenal administration.

## Claims (Claims for the following Contracting State(s): AT, ES, GR)

1. A method for the preparation of a composition comprising the compounds 2,4-diamino-5-(8-dimethylamino-7-methyl-5-quinolylmethyl)pyrimidine and sulphadimidine, or salts thereof, which method comprises bringing into association the 2,4-diamino-5-(8-dimethylamino-7-methyl-5-quinolylmethyl)pyrimidine and the sulphadimidine.

2. A method according to claim 1 wherein the said compounds are present in a ratio of between 1:1 and 1:20.

3. A method according to claim 1 wherein the composition is for parenteral administration and the compounds are present in the ratio of 1:5.

4. A method according to claim 1 wherein the composition is a bolus or drench composition and the compounds are present in the ratio of 1:9.

5. A method according to claim 1 wherein the composition is a paste or infeed composition and the compounds are present in the ratio of 1:5.

6. A method according to claim 1 wherein the composition is for the treatment of bacterial infections in animals.

7. A method according to claim 6 wherein the composition is for the treatment of bacterial infections in animals by oral administration.

8. A method according to claim 6 wherein the composition is for the treatment of bacterial infections in animals by intraruminal administration.

9. A method according to claim 1 wherein the composition is a bolus which contains from 1 to 8 mg/kg of 2,4-diamino-5-(8-dimethylamino-7-methyl-5-quinolylmethyl)pyrimidine and from 9 to 72 mg/kg of sulphadimidine.

10. A method according to claim 1 wherein the composition is a bolus which contains from 6 to 16 mg/kg of 2,4-diamino-5-(8-dimethylamino-7-methyl-5-quinolylmethyl)pyrimidine and from 54 to 144 mg/kg of sulphadimidine.

11. A method according to claim 1 wherein the composition is a bolus which contains 8 mg/kg of 2,4-diamino-5-(8-dimethylamino-7-methyl-5-quinolylmethyl) pyrimidine and 72 mg/kg sulphadimidine.

12. A composition which comprises the compounds 2,4-diamino-5-(8-dimethylamino-7-methyl-5-quinolylmethyl)pyrimidine and sulphadimidine for use in the manufacture of a medicament.

13. A composition according to claim 12 which comprises the compounds for use in the manufacture of a medicament for oral administration.

14. A composition according to claim 12 which comprises the compounds for use in the manufacture of a medicament for intrarumenal administration.

15. A composition whenever prepared by the method of any one of claims 1 to 5 or 9 to 11.

## Patentansprüche (Patentansprüche für folgende(n) Vertragsstaat(en): BE, CH, DE, FR, GB, IT, LI, LU, NL, SE)

1. Zusammensetzung, enthaltend die Verbindungen 2,4-Diamino-5-(8-dimethylamino-7-methyl-5-chinolylmethyl)pyrimidin und Sulfadimidin oder deren Salze.

2. Zusammensetzung gemäß Anspruch 1, in der die Verbinddungen in einem Verhältnis zwischen 1:1 und 1:20 vorliegen.

3. Zusammensetzung gemäß Anspruch 1 für die parenterale Verabreichung, in der die Verbindungen in dem Verhältnis 1:5 vorliegen.

4. Bolus- oder Arzneitrank-Zusammensetzung gemäß Anspruch 1, in der die Verbindungen in dem Verhältnis 1:9 vorliegen.

5. Pastenzusammensetzung oder in der Nahrung zu verabreichende Zusammensetzung gemäß Anspruch 1, in der die Verbindungen in dem Verhältnis 1:5 vorliegen.

6. Zusammensetzung gemäß einem der Ansprüche 1 bis 5 für die Verwendung bei der Behandlung von bakteriellen Infektionen bei Tieren.

7. Zusammensetzung gemäß Anspruch 6 für die Verwendung bei der Behandlung von bakteriellen Infektionen bei Tieren durch orale Verabreichung.

8. Zusammensetzung gemäß Anspruch 6 für die Verwendung bei der Behandlung von bakteriellen Infektionen bei Tieren durch Verabreichung innerhalb des Pansens.

9. Zusammensetzung gemäß Anspruch 1 in Form eines Bolus, der 1 bis 8 mg/kg 2,4-Diamino-5-(8-dimethylamino-7-methyl-5-chinolylmethyl)-pyrimidin und 9 bis 72 mg/kg Sulfadimidin enthält.

10. Zusammensetzung gemäß Anspruch 1 in Form eines Bolus, der 6 bis 16 mg/kg 2,4-Diamino-5-(8-dimethylamino-7-methyl-5-chinolylmethyl)-pyrimidin und 54 bis 144 mg/kg Sulfadimidin enthält.

11. Zusammensetzung gemäß Anspruch 1 in Form eines Bolus, der 8 mg/kg 2,4-Diamino-5-(8-dimethylamino-7-methyl-5-chinolylmethyl)-pyrimidin und 72 mg/kg Sulfadimidin enthält.

12. Zusammensetzung, enthaltend die Verbindungen 2,4-Diamino-5-(8-dimethylamino-7-methyl-5-chinolylmethyl)pyrimidin und Sulfadimidin zur Verwendung bei der Herstellung eines Arzneimittels.

13. Zusammensetzung gemäß Anspruch 12, enthaltend die Verbindungen zur Verwendung bei der Herstellung eines Arzneimittels für die orale Verabreichung.

14. Zusammensetzung gemäß Anspruch 12, enthaltend die Verbindungen für die Verwendung bei der Herstellung eines Arzneimittels für die Verabreichung innerhalb des Pansens.

## Patentansprüche (Patentansprüche für folgende(n) Vertragsstaat(en): AT, ES, GR)

1. Verfahren zur Herstellung einer Zusammensetzung, enthaltend die Verbindungen 2,4-Diamino-5-(8-dimethylamino-7-methyl-5-chinolylmethyl)-pyrimidin und Sulfadimidin oder deren Salze, bei dem man das 2,4-Diamino-5-(8-dimethylamino-7-methyl-5-chinolylmethyl)-pyrimidin und das Sulfadimidin assoziiert.

2. Verfahren gemäß Anspruch 1, bei dem die Verbindungen in einem Verhältnis zwischen 1:1 und 1:20 vorliegen.

3. Verfahren gemäß Anspruch 1, bei dem die Zusammensetzung für die parenterale Verabreichung bestimmt ist, und die Verbindungen im Verhältnis 1:5 vorliegen.

4. Verfahren gemäß Anspruch 1, bei dem die Zusammensetzung eine Bolus- oder Arzneitrank-Zusammensetzung ist, und die Verbindungen im Verhältnis 1:9 vorliegen.

5. Verfahren gemäß Anspruch 1, bei dem die Zusammensetzung eine Pastenzusammensetzung oder eine Zusammensetzung für die Verabreichung in der Nahrung ist, und die Verbindungen im Verhältnis 1:5 vorliegen.

6. Verfahren gemäß Anspruch 1, bei dem die Zusammensetzung für die Behandlung von bakteriellen Infektionen bei Tieren bestimmt ist.

7. Verfahren gemäß Anspruch 6, bei dem die Zusammensetzung für die Behandlung von bakteriellen Infektionen bei Tieren durch orale Verabreichung bestimmt ist.

8. Verfahren gemäß Anspruch 6, bei dem die Zusammensetzung für die Behandlung von bakteriellen Infektionen bei Tieren durch Verabreichung innerhalb des Pansens bestimmt ist.

9. Verfahren gemaß Anspruch 1, bei dem die Zusammensetzung ein Bolus ist, der 1 bis 8 mg/kg 2,4-Diamino-5-(8-dimethylamino-7-methyl-5-chinolylmethyl)-pyrimidin und 9 bis 72 mg/kg Sulfadimindin enthält.

10. Verfahren gemäß Anspruch 1, bei dem die Zusammensetzung ein Bolus ist, der 6 bis 16 mg/kg 2,4-Diamino-5-(8-dimethylamino-7-methyl-5-chinolylmethyl)-pyrimidin und 54 bis 144 mg/kg Sulfadimidin enthält.

11. Verfahren gemäß Anspruch 1, bei dem die Zusammensetzung ein Bolus ist, der 8 mg/kg 2,4-Diamino-5-(8-dimethylamino-7-methyl-5-chinolylmethyl)-pyrimidin und 72 mg/kg Sulfadimidin enthält.

12. Zusammensetzung, enthaltend die Verbindungen 2,4-Diamino-5-(8-dimethylamino-7-methyl-5-chinolylmethyl)pyrimidin und Sulfadimidin zur Verwendung bei der Herstellung eines Arzneimittels.

13. Zusammensetzung gemäß Anspruch 12, enthaltend die Verbindungen zur Verwendung bei der Herstellung eines Arzneimittels für die orale Verabreichung.

14. Zusammensetzung gemäß Anspruch 12, enthaltend die Verbindungen zur Verwendung bei der Herstellung eines Arzneimittels für die Verabreichung innerhalb des Pansens.

15. Zusammensetzung, hergestellt nach dem Verfahren gemäß einem der Ansprüche 1 bis 5 oder 9 bis 11.

## Revendications (Revendications pour l'(les) Etat(s) contractant(s) suivant(s): BE, CH, DE, FR, GB, IT, LI, LU, NL, SE)

1. Composition comprenant les composés 2,4-diamino-5-(8-diméthylamino-7-méthyl-5-quinolylméthyl) pyrimidine et sulfadimidine, ou leurs sels.

2. Composition selon la revendication 1, dans laquelle lesdits composés sont présents en un rapport compris entre 1:1 et 1:20.

3. Composition selon la revendication 1, pour l'administration parentérale, dans laquelle les composés sont présents en un rapport de 1:5.

4. Composition sous forme de bolus ou de dose selon la revendication 1, dans laquelle les composés sont présents en un rapport de 1:9.

5. Composition en pâte ou pour incorporation dans la nourriture selon la revendication 1, dans laquelle les composés sont présents en un rapport de 1:5.

6. Composition selon l'une quelconque des revendications 1 à 5 pour utilisation dans le traitement des infections bactériennes chez l'animal.

7. Composition selon la revendication 6 pour utilisation dans le traitement des infections bactériennes chez l'animal par administration orale.

8. Composition selon la revendication 6 pour utilisation dans le traitement des infections bactériennes chez l'animal par administration intraruménale.

9. Composition selon la revendication 1 sous la forme d'un bolus qui contient de 1 à 8 mg/kg de 2,4-diamino-5-(8-diméthylamino-7-méthyl-5-quinolylméthyl) pyrimidine et de 9 à 72 mg/kg de sulfadimidine.

10. Composition selon la revendication 1 sous forme d'un bolus qui contient de 6 à 16 mg/kg de 2,4-diamino-5-(8-diméthylamino-7-méthyl-5-quinolylméthyl) pyrimidine et de 54 à 144 mg/kg de sulfadimidine.

11. Composition selon la revendication 1 sous forme d'un bolus qui contient 8 mg/kg de 2,4-diamino-5-(8-diméthylamino-7-méthyl-5-quinolylméthyl) pyrimidine et 72 mg/kg de sulfadimidine.

12. Composition qui comprend les composés 2,4-diamino-5-(8-diméthylamino-7-méthyl-5-quinolylméthyl) pyrimidine et sulfadimidine pour l'utilisation dans la fabrication d'un médicament.

13. Composition selon la revendication 12 qui comprend les composés pour l'utilisation dans la fabrication d'un médicament pour une administration orale.

14. Composition selon la revendication 12 qui comprend les composés pour l'utilisation dans la fabrication d'un médicament pour une administration intraruménale.

## Revendications (Revendications pour l'(les) Etat(s) contractant(s) suivant(s): AT, ES, GR)

1. Procédé de préparation d'une composition comprenant les composés : 2,4-diamino-5-(8-diméthylamino-7-méthyl-5-quinolylméthyl) pyrimidine et sulfadimidine, ou leurs sels, lequel procédé comprend la mise en association de la 2,4-diamino-5-(8-diméthylamino-7-méthyl-5-quinolylméthyl) pyrimidine et de la sulfadimidine.

2. Procédé selon la revendication 1, dans lequel lesdits composés sont présents en un rapport compris entre 1:1 et 1:20.

3. Procédé selon la revendication 1, dans lequel la composition est destinée à l'administration parentérale et les composés sont présents en un rapport de 1:5.

4. Procédé selon la revendication 1, dans lequel la composition est sous la forme d'un bolus ou d'une dose et les composés sont présents en un rapport de 1:9.

5. Procédé selon la revendication 1, dans lequel la composition est une pâte ou destinée à être incorporée dans la nourriture et les composés sont présents en un rapport de 1:5.

6. Procédé selon la revendication 1, dans lequel la composition est destinée au traitement des infections bactériennes chez l'animal.

7. Procédé selon la revendication 6, dans lequel la composition est destinée au traitement des infections bactériennes chez l'animal par administration orale.

8. Procédé selon la revendication 6, dans lequel la composition est destinée au traitement des infections bactériennes chez l'animal par administration intraruménale.

9. Procédé selon la revendication 1, dans lequel la composition est sous la forme d'un bolus qui contient de 1 à 8 mg/kg de 2,4-diamino-5-(8-diméthylamino-7-méthyl-5-quinolylméthyl) pyrimidine et de 9 à 72 mg/kg de sulfadimidine.

10. Procédé selon la revendication 1, dans lequel la composition est sous la forme d'un bolus qui contient de 6 à 16 mg/kg de 2,4-diamino-5-(8-diméthylamino-7-méthyl-5-quinolylméthyl) pyrimidine et de 54 à 144 mg/kg de sulfadimidine.

11. Procédé selon la revendication 1, dans lequel la composition est sous la forme d'un bolus qui contient 8 mg/kg de 2,4-diamino-5-(8-diméthylamino7-méthyl-5-quinolylméthyl) pyrimidine et 72 mg/kg de sulfadimidine.

12. Composition qui comprend les composés : 2,4-diamino-5-(8-diméthylamino-7-méthyl-5-quinolylméthyl) pyrimidine et sulfadimidine pour l'utilisation dans la fabrication d'un médicament.

13. Composition selon la revendication 12 qui comprend les composés pour l'utilisation dans la fabrication d'un médicament pour une administration orale.

14. Composition selon la revendication 12 qui comprend les composés pour l'utilisation dans la fabrication d'un médicament pour une administration intrarumérale.

15. Composition préparée par un procédé selon l'une quelconque des revendications 1 à 5 ou 9 à 11.
